# EUROPEAN PATENT SPECIFICATION

(11) **EP 3 833 399 B1**
(45) Date of publication and mention of the grant of the patent: **08.04.2026**
(21) Application number: 19846374.7
(22) Date of filing: 08.08.2019
(51) Int. Cl.: A61K 49/00, A61K 49/14, A61K 38/08, G01N 33/574

(54) **BINDING MOLECULES TO TUMOR ASSOCIATED MACROPHAGES AND METHODS OF USE**
BINDEMOLEKÜLE AN TUMORASSOZIIERTE MAKROPHAGEN UND VERFAHREN ZUR VERWENDUNG
MOLÉCULES DE LIAISON À DES MACROPHAGES ASSOCIÉS À UNE TUMEUR ET PROCÉDÉS D'UTILISATION

(30) Priority: 10.08.2018 US 201862717656 P
(43) Date of publication of application: 16.06.2021
(73) Proprietor: Sanford Burnham Prebys Medical Discovery Institute, La Jolla, CA 92037 (US)
(72) Inventor: PANG, Hongbo, La Jolla, California 92037 (US)
(74) Representative: Dehns
(86) International application number: PCT/US2019/045676
(87) International publication number: WO 2020/033663

(56) References cited:
- US-A- 5 399 586
- US-A1- 2014 286 973
- US-A1- 2014 286 973
- US-A1- 2015 018 526
- US-A1- 2017 266 303
- US-A1- 2017 266 303
- BYUNGJI KIM ET AL: "Immunogene therapy with fusogenic nanoparticles modulates macrophage response to Staphylococcus aureus", NATURE COMMUNICATIONS, vol. 9, no. 1, 17 May 2018 (2018-05-17), pages 1969, XP055691105, DOI: 10.1038/s41467-018-04390-7
- XIANLEI SUN ET AL: "Molecular Imaging of Tumor-Infiltrating Macrophages in a Preclinical Mouse Model of Breast Cancer", THERANOSTICS, vol. 5, no. 6, 1 January 2015 (2015-01-01), AU, pages 597 - 608, XP055446955, ISSN: 1838-7640, DOI: 10.7150/thno.11546
- PABLO SCODELLER ET AL: "Precision Targeting of Tumor Macrophages with a CD206 Binding Peptide", SCIENTIFIC REPORTS, vol. 7, no. 1, 7 November 2017 (2017-11-07), XP055690885, DOI: 10.1038/s41598-017-14709-x
- BIANCA KAKOSCHKY ET AL: "Selective targeting of tumor associated macrophages in different tumor models", PLOS ONE, vol. 13, no. 2, 15 February 2018 (2018-02-15), XP055701870, DOI: 10.1371/journal.pone.0193015
- WNUK A ET AL: "The Crucial Involvement of Retinoid X Receptors in DDE Neurotoxicity", NEUROTOXICITY RESEARCH, HARWOOD ACADEMIC PUBLISHERS, LAUSANNE, CH, vol. 29, no. 1, 13 November 2015 (2015-11-13), pages 155 - 172, XP035962629, ISSN: 1029-8428, [retrieved on 20151113], DOI: 10.1007/S12640-015-9572-6
- KIM ET AL.: "Immunogene Therapy With Fusogenic Nanoparticles Modulates Macrophage Response To Staphylococcus Aureus", NATURE COMMUNACATIONS, vol. 9, 17 May 2018 (2018-05-17), pages 1969, XP055691105
- TANG ET AL.: "Tumor-specific Macrophage Targeting Through Recognition of Retinoid X Receptor Beta", JOURNAL OF CONTROLLED RELEASE, vol. 301, 2019, pages 42 - 53, XP085672788, DOI: 10.1016/j.jconrel.2019.03.009

## Description

This invention was made with government support under R21 EB022652 awarded by the National Institutes of Health (NIH). The government has certain rights in the invention.

### BACKGROUND OF THE INVENTION

Macrophages are distributed across all major organs, play central roles in normal immune homeostasis and disease progression, such as cancer, and are key regulators in the tumor microenvironment. In many tumors, anti-inflammatory macrophages (alternatively activated or M2 subtype), also known as tumor-associated macrophages (TAMs), are responsible for generating an immunosuppressive tumor microenvironment, which prevents the recognition and elimination of tumor cells by the immune system.

Kakoschky et al. 2018 (PLoS ONE 13(2): e0193015) describes a peptide, termed M2pep, that selectively binds to, and is internalized by, *in vitro*-differentiated murine M2 macrophages as compared to M1 macrophages and that could selectively target TAMs in HCC and breast carcinoma. Schodeller et al., 2017 (Scientific Reports, 7: 14655) describes an *in vivo* peptide phage display screen in mice bearing 4T1 metastatic breast tumors to identify peptides that target peritoneal macrophages and describes a peptide, termed UNO, that selectively targets MRC1-expressing TAMs (MEMs) and interacts with recombinant CD206. Sun et al., 2015 (Theranostics, Vol. 5, Issue 5, pp. 597-608) describes the use of a CD206-targeting near-infrared fluorescence (NIRF) agent, Dye-anti-CD206, in NIRF imaging to noninvasively image tumor-infiltrating macrophages in the 4T1 mouse breast cancer model. Kim et al., 2018 (Nature Communications, Vol. 9 (1), p. 1969) describes the use of porous silicon nanoparticles containing an outer sheath of homing peptides and fusogenic liposome to selectively target macrophages and directly introduce an oligonucleotide payload into the cytosol to knockdown of the proinflammatory macrophage marker IRF5. US 2014/0286973 discloses methods for treating leukemia, such as acute myeloid leukemia (AML), using T cells expressing a chimeric antigen receptor (CAR) specific to folate receptor beta (FRβ) which may be administered with an RXR agonist. US 2017/0266303 describes methods for treating cancers by targeting tumor associated macrophages using folate receptor binding ligands attached to a drug.

### SUMMARY OF THE INVENTION

Described herein are tumor associated macrophage (TAM) binding molecules for use in methods of treating or diagnosing the cancer, the methods comprising administering a pharmaceutical composition comprising a tumor associated macrophage (TAM) binding molecule conjugated to a therapeutic agent or label, respectively, wherein the TAM binding molecule binds to retinoid X receptor beta on the TAM, and wherein the TAM binding molecule is an anti-RXR antibody, or antigen-binding fragment thereof, or the TAM binding molecule is CRVLRSGSC. The pharmaceutical composition may be cytolytic to TAMs, may remove, reduce and/or neutralize TAMs, or may repolarize TAMs from an M2 phenotype to an M1 phenotype.

In some embodiments, the TAM binding molecule is an anti-RXR antibody or antigen-binding fragment thereof and may be: an IgG, IgA, or IgM antibody; a single domain antibody; or a chimeric, humanized, or human antibody. The antigen binding fragment may be a Fab, Fab', Fab'-SH, Fv, scFv, F(ab')2, or a diabody. The TAM binding molecule may be the peptide CRVLRSGSC, which is cyclic. In some instances, the therapeutic agent is a cytotoxic agent, a chemotherapeutic agent, a protein, a peptide, an antibody, a growth inhibitory agent, a nucleic acid, or an anti-hormonal agent. In other instances, the therapeutic agent is a cytotoxic agent wherein the cytotoxic agent is a ribosome inactivating protein, a histone deacetylase (HDAC) inhibitor, a tubulin inhibitor, an alkylating agent, an antibiotic, an antineoplastic agent, an antiproliferative agent, an antimetabolite, a topoisomerase I or II inhibitor, a hormonal agonist or antagonist, an immunomodulator, a DNA minor groove binder, or a radioactive agent. In some instances, the label is a fluorescent label, a chromogenic label, or a radiolabel. The TAM binding molecule may be directly conjugated to the therapeutic agent or label. In other embodiments, the TAM binding molecule may be indirectly conjugated to the therapeutic agent or label via a linker. A delivery agent may be a liposome, microsphere, nanoparticle, microemulsion, microcapsule, polymer matrix, hydrogel, or viral vector.

The cancer may be selected from the group consisting of brain cancer, renal cancer, ovarian cancer, prostate cancer, lymphoma, breast cancer colon cancer, lung cancer, squamous cell carcinoma of the head and neck, and melanoma. The pharmaceutical composition may be administered topically, subcutaneously, intravenously, intradermally, intraperitoneally, orally, intramuscularly, or intracranially. The pharmaceutical composition may be administered in combination with a second therapeutic agent. The second therapeutic agent may be a cancer chemotherapeutic agent, radiation therapy, a cytotoxic agent, another antibody, a NSAID, a corticosteroid, a dietary supplement (e.g. an antioxidant), or a combination thereof.

### BRIEF DESCRIPTION OF THE DRAWINGS

A better understanding of the features and advantages of the present invention will be obtained by reference to the following detailed description that sets forth illustrative embodiments, in which the principles of the invention are utilized, and the accompanying drawings of which:
TABLE 1 is a summary of tumors tested for CRV in vivo homing.

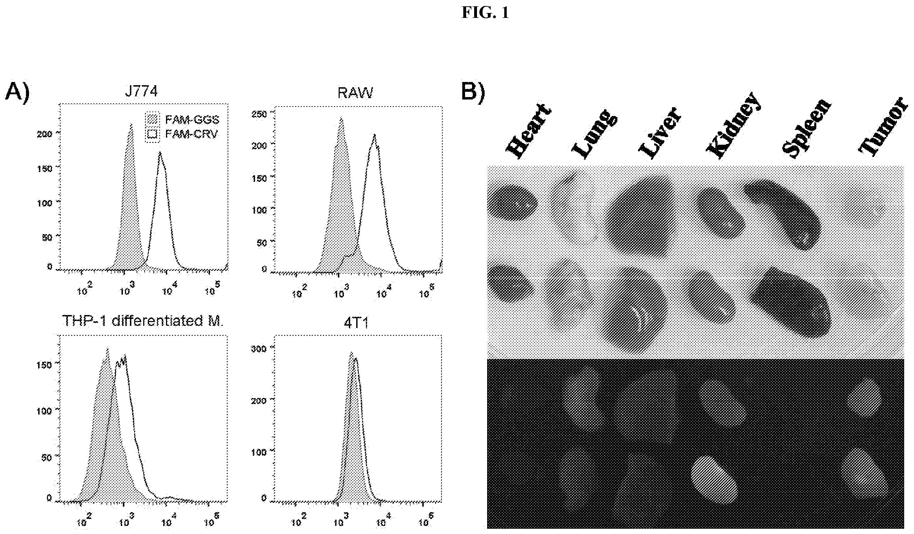

**FIG 1** is a depiction of FAM-peptide binding. **A)** *In vitro* binding of FAM-peptide to different cell lines. Cells were incubated with FAM-CRV or FAM-GGS (10 µM) at 4 °C for 1 h and washed with PBS. Fluorescence was measured by flow cytometry. High binding of FAM-CRV compared to FAM-GGS was found on J774, RAW, and THP-1 differentiated macrophages, but not on 4T1 tumor cells. **B)** Representative UV illumination image of FAM-CRV homing in different tissues in 4T1 breast cancer mice. 100 µg of FAM-CRV in 100 µL PBS was injected intravenously into 4T1 mice for 1-h circulation. Tissues were collected after transcardial perfusion with PBS.

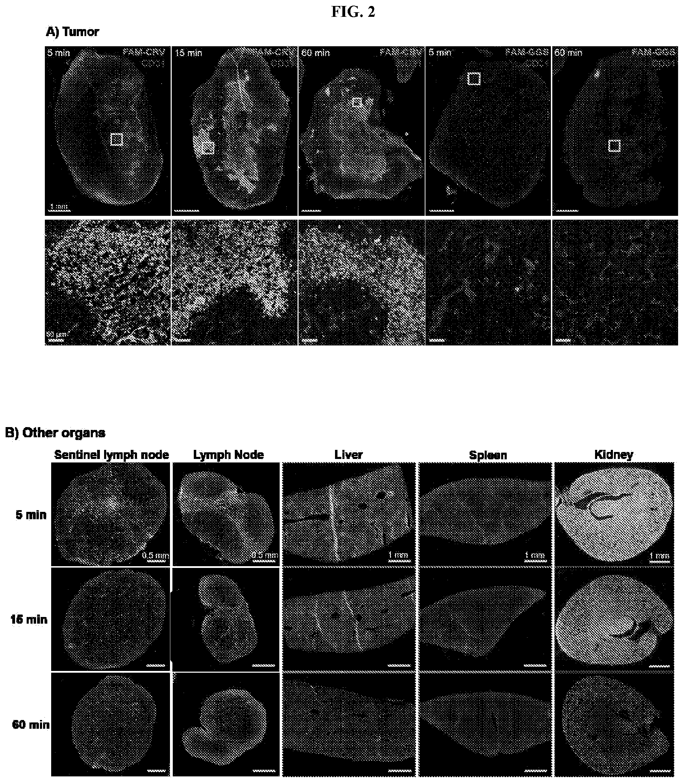

**FIG 2** depicts immunofluorescence staining of tissues for FAM-peptide and CD31 with different homing time points (5, 15, and 60 min). Tissue sections of 4T1 orthotopic tumor-bearing mice injected with FAM-CRV or FAM-GGS peptide were stained with anti-FITC, anti-CD31 antibodies, and DAPI. **A)** Homing results of FAM-CRV or FAM-GGS in tumors at different time points. Top row: whole tumor view. Bottom row: magnified view of regions marked by white squares in top row. **B)** Distribution of FAM-CRV in other organs. FAM-CRV signal washed out with time.

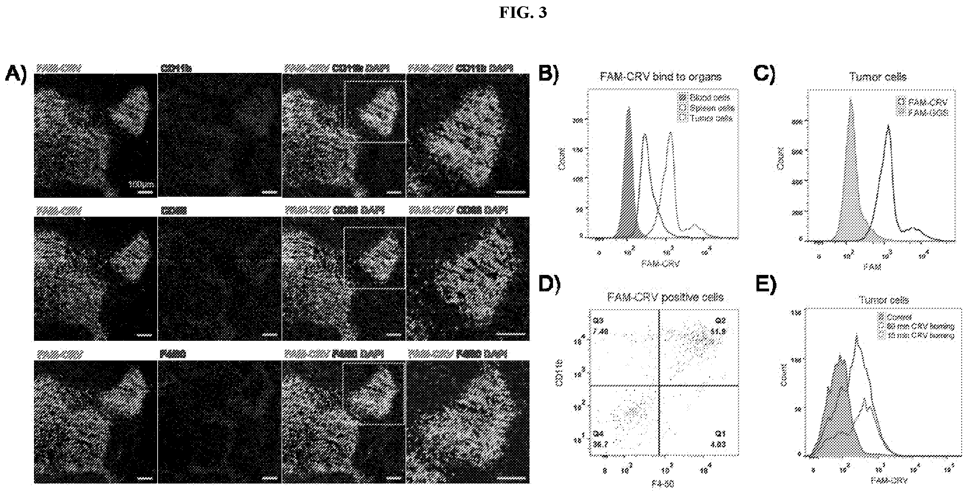

**FIG 3** depicts the association of FAM-CRV with tumor macrophages. **A)** Immunofluorescence staining of 4T1 mouse tissues for FAM-CRV and macrophage markers. Tissue sections were stained with anti-FITC, anti-CD11b, anti-F4/80 or anti-CD68 antibodies, and DAPI . Scale bars: 100 µm. **B-D)** *Ex vivo* binding of FAM-CRV or FAM-GGS to cells isolated from different organs of 4T1 mice. Cells were incubated with FAM-CRV or FAM-GGS, CD11b, CD68, and F4/80 primary and secondary antibodies at 4 °C for 1 h. The cells were then washed and analyzed by flow cytometry. B: Preferential binding of FAM-CRV to tumor cells compared to blood and spleen cells. C: FAM-CRV or FAM-GGS binding to tumor cells. D: Population of CD11b and F4-80 positive cells in FAM-CRV positive cells. **E)** Flow cytometric analysis of cells isolated from 4T1 tumors after *in vivo* FAM-CRV homing. Tumors were obtained at 15 or 60 min post intravenous administration of FAM-CRV. Cells were incubated with CD11b, and F4/80 primary and secondary antibodies at 4 °C for 1 h, washed, and analyzed with flow cytometry.

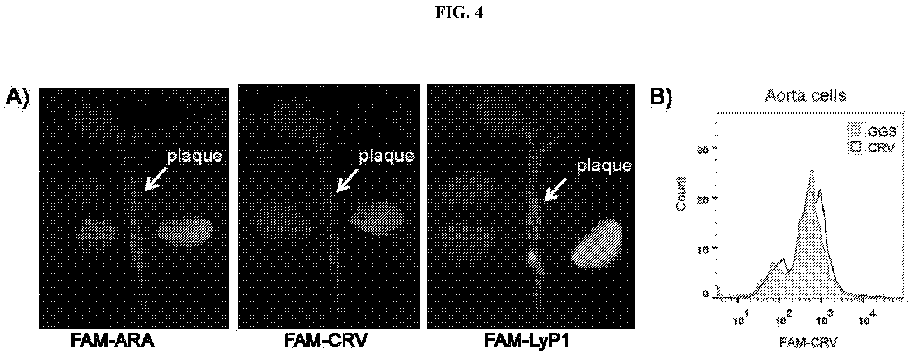

**FIG 4** depicts the evaluation of FAM-CRV binding to macrophages in atherosclerotic plaques. **A)** *In vivo* homing of FAM-CRV, FAM-ARA (negative control), or FAM-LyP-1 (positive control) in ApoE -/- mice bearing atherosclerotic plaques in the aorta. 100 µg of the FAM-CRV, FAM-LyP1, or FAM-ARA was injected intravenously for 1-h circulation. The aorta (middle), kidney (right) and liver (left) were excised and imaged with UV illuminator. **B)** *Ex vivo* staining of isolated from aorta with plaques with FAM-CRV or FAM-GGS. Cells were incubated at 4 °C for 1 h, and then analyzed by flow cytometry.

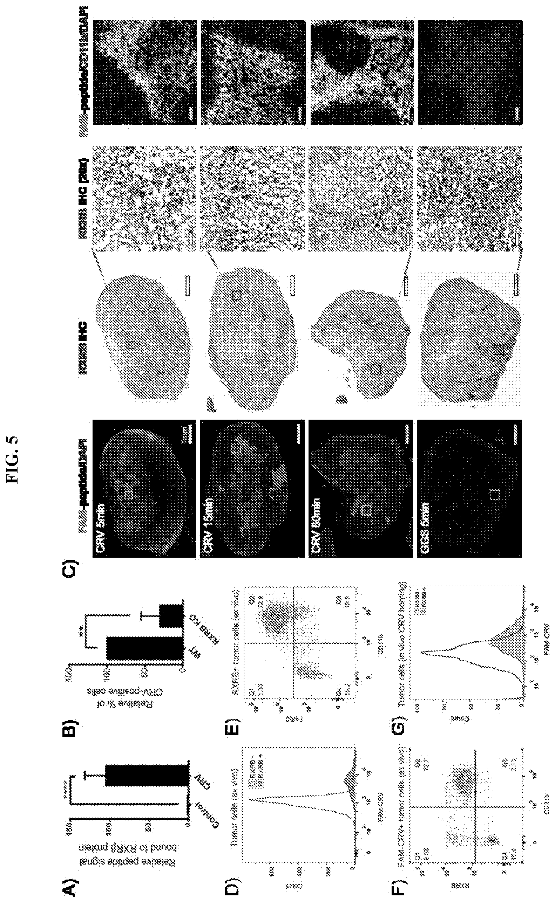

**FIG 5** depicts the evaluation of RXRB as a CRV binding receptor. **A)** High binding of FAM-CRV compared to FAM-GGS to immobilized recombinant human RXRB protein. **B)** Silencing the expression of RXRB inhibited CRV binding to RAW at 4 °C for 1 h. **C)** Immunohistochemical staining of RXRB on 4T1 tumors with FAM-CRV or FAM-GGS *in vivo* homing. RXRB IHC staining colocalized with FAM-CRV and CD11b from immunofluorescence staining. **D-F)** *Ex vivo* binding of CRV to dissociated cells from 4T1 tumor. Cells were stained with FAM-CRV or FAM-GGS, CD11b, CD68, F4/80, and RXRB primary and secondary antibodies at 4 °C for 1 h. The cells were washed and analyzed with flow cytometry. **G)** Cell staining after *in vivo* FAM-CRV homing. Tumors were obtained at 60 min post intravenous administration of FAM-CRV. Cells were incubated with RXRB, CD11b, and F4/80 primary and secondary antibodies at 4 °C for 1 h. The cells were washed and analyzed with flow cytometry.

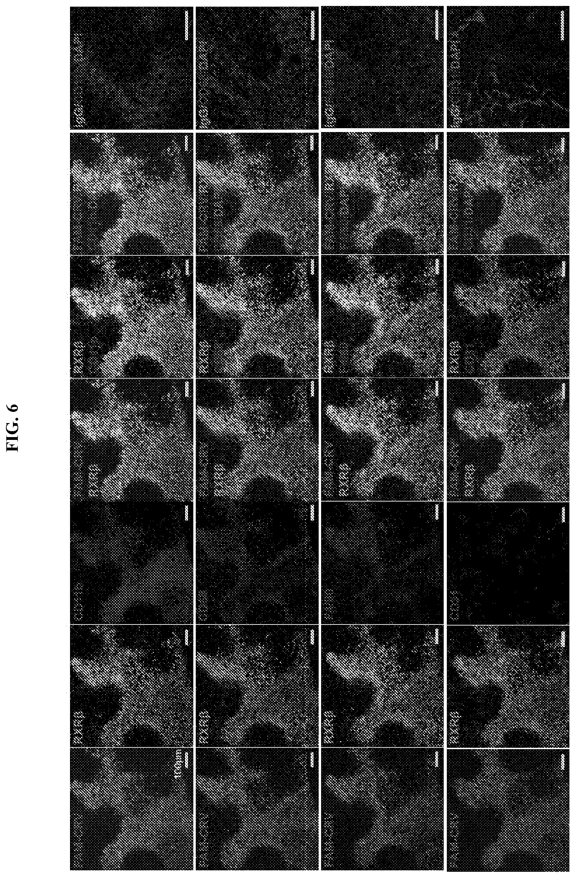

**FIG 6** depicts immunofluorescence staining of 4T1 mouse tissues with RXRB antibody and FAM-CRV *in vivo* homing. Tissue sections of 4T1 orthotopic tumor-bearing mice were stained with anti-FITC, anti-RXRB antibodies or control rabbit IgG, anti-CD11b, CD68, F4/80, or CD31 antibodies and DAPI. The last column shows a merged image from the control IgG-receiving animals.

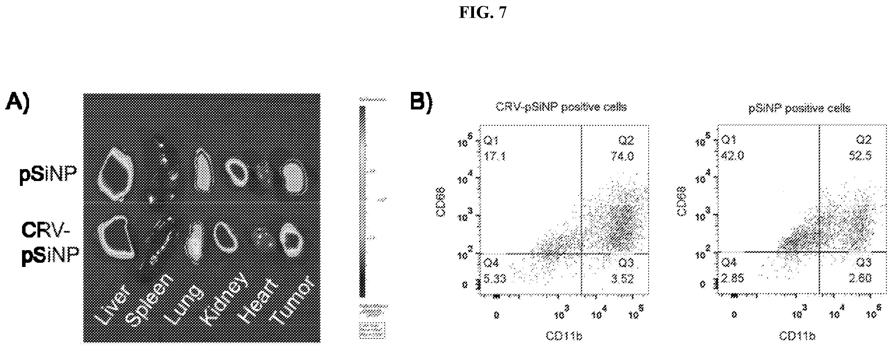

**FIG 7** describes CRV-pSiNP homing. **A)** Fluorescence imaging of tissues from 4T1 mice after 1-h circulation of NPs. Top row: mouse injected with pSiNP. Bottom row: mouse injected with CRV-pSiNP. **B)** Flow cytometric analysis of cells isolated from 4T1 tumors after *in vivo* CRV-pSiNP or pSiNP homing. Tumors were obtained 60 min post intravenous administration. Cells were incubated with CD11b and CD68 primary and secondary antibodies at 4 °C for 1 h, washed, and analyzed with flow cytometry.

### DETAILED DESCRIPTION OF THE INVENTION

### Macrophages and the Tumor Microenvironment

In vivo, the tumor microenvironment is a complex milieu containing multiple cell types including tumor cells, vascular cells such as endothelial cells, and stromal cells, such as fibroblasts. In addition, in vivo, these cells are exposed to blood flow and various biological transport conditions. In vivo, microvascular cells in a tumor are affected by blood flow and communicate with tumor and non-tumor cells, both physically and through diffusible factors. In addition, the tumor vasculature is abnormal, characterized by chaotic branching, a low flow rate, and leaky vessels, and thus serves as a major transport barrier to anticancer therapies that target tumor cells. The interplay between tumor cells, endothelial cells, and stromal cells affects each cell type, leading to increased angiogenesis and tumor cell proliferation, and this crosstalk may be an important factor in determining the responsiveness of tumor cells to anticancer drugs.

In the tumor microenvironment cells experience oxygen and nutrient deprivation. Hypoxic stress alters the metabolism of tumor cells and macrophages within tumors with subsequent changes in the microenvironment. The change in microenvironment alters the phenotype and metabolism of macrophages to induce a tumor-promoting reprogramming. Nutrient stress also provokes autophagy to guarantee cell survival or to induce cell death. Death of tumor cells is communicative system attracting macrophages and directing their phenotype. Depending on the mode of tumor cell death macrophage polarization ranges from proinflammatory activation to anti-inflammatory/immuno-suppressive activation.

Chronic inflammation contributes to cancer development. The presence and activation of chronic innate immune cell types (e.g., neutrophils, macrophages, and mast cells) promote cancer development. Thus it is clear that some subsets of chronically activated innate cells promote the growth and/or facilitate the survival of neoplastic cells. Depending on their polarization status, immune cells can exert either antitumor (e.g. T-helper 1 (Th1) vs Th17 subsets of CD4(+) T cells, type I) or protumor (e.g. vs type II NKT cells, M1 vs M2 macrophages, and N1 vs N2 neutrophils) functions. Chronically activated and polarized immune cells (e.g. M2 macrophages and N2 neutrophils) produce or carry a myriad of chemokines, cytokines, growth factors, and proteases leading to tissue remodeling, angiogenesis, cell proliferation, genomic instability, and expansion of neoplastic cells into ectopic tissue.

Macrophages are a type of white blood cell, of the immune system, that engulfs and digests cellular debris, foreign substances, microbes, cancer cells, and anything else that does not have the type of proteins specific to healthy body cells on its surface in a process called phagocytosis. Macrophages increase inflammation and stimulate the immune system. Macrophages also play an important anti-inflammatory role and can decrease immune reactions through the release of cytokines. Macrophages that encourage inflammation are called M1 macrophages, whereas macrophages that decrease inflammation and encourage tissue repair are called M2 macrophages. Macrophages are a dominating immune cell population in most solid tumors. Tumor-associated macrophages (TAMs) are a type of macrophage. TAMs are thought to acquire an M2 phenotype, contributing to tumor growth and progression. TAMs can regulate tumor progression. Therapeutic strategies to reduce the number of TAMs or to manipulate the TAM phenotype are of interest to cancer therapy.
Retinoid X receptors (RXR) are members in the superfamily of nuclear receptor and have essential nuclear and cytoplasmic functions as transcription factors. RXR is a type of nuclear receptor that is activated by 9-cis retinoic acid, which is contemplated to be of endogenous relevance. RXR is also activated by 9-cis-13,14-dihydro-retinoic acid, may be the major endogenous mammalian RXR-selective agonist. There are three retinoic X receptors (RXR): RXR-alpha (RXRA), RXR-beta (RXRB), and RXR-gamma (RXRG), encoded by the *RXRA, RXRB, RXRG* genes, respectively. RXR is a hetero-dimerization partner for the members of the subfamily II nuclear receptors which regulate the transcription of numerous target genes, following chemical activation. RXR can heterodimerize with subfamily I nuclear receptors including CAR, FXR, LXR, PPAR, PXR, RAR, TR, and VDR. The RXR heterodimer in the absence of ligand is bound to hormone response elements complexed with corepressor protein. Binding of agonist ligands to RXR results in dissociation of corepressor and recruitment of coactivator protein. This promotes transcription of the downstream target gene into mRNA and eventually protein. RXR can regulate macrophages in inflammatory and metabolic disorders and there is potential for direct modulation of RXR signaling to treat macrophage-related pathologies. The cell surface expression of RXRB is specific to TAMs and RXRB is responsible for the preferential binding of CRV and other contemplated molecules to tumor macrophages.

### Molecules that Target TAMs

The peptide CRV (CRVLRSGSC) is a cyclic macrophage-targeting peptide with a disulfide bond between the terminal cysteine residues. CRV selectively homes to tumors and binds with TAMs within said tumors. CRV recognizes and binds to RXRB on the surface of TAMs within tumors. CRV only recognizes TAMs, but not macrophages in atherosclerotic plaques.

CRV can be linked to a therapeutic agent. The therapeutic agent can comprise antibodies (e.g. IgG, IgA, or IgM).

CRV can be modified to produce a related peptide which is contemplated to also act as a TAM molecule. Examples of such a modification include one or more substitutions, deletions, or additions of amino acids. Conservative substitutions include amino acid substitutions that substitute a given amino acid with another amino acid of similar characteristics and further include, among the aliphatic amino acids interchange of alanine, valine, leucine, and isoleucine; interchange of the hydroxyl residues serine and threonine, exchange of the acidic residues aspartate and glutamate, substitution between the amide residues asparagine and glutamine, exchange of the basic residues lysine and arginine, and replacements among the aromatic residues phenylalanine and tyrosine.

Other TAM binding molecules which can act as a cell surface RXRB binding molecule to selectively bind TAMs comprise antibodies (e.g. IgG, IgA, or IgM), antigen binding fragments, peptides, ligands, non-IG domains, or small molecules. Said TAM binding molecules may bind RXRB at different locations on the RXRB molecule. If the TAM binding molecule is an antibody, it can be a single domain antibody, chimeric antibody, humanized antibody, human antibody, and/or monoclonal antibody. If the TAM binding molecule is an antigen binding fragment, it can be a Fab, Fab', Fab'-SH, Fv, scFv, F(ab')2, or a diabody. The TAM binding molecule may be CRV.

Modifications to the TAM binding molecules are contemplated. These modifications include conjugation to an additional moiety. This moiety can be a therapeutic agent or a diagnostic agent to be used clinically or for research purposes.

These modifications can further constitute a delivery system, which can deliver the TAM binding molecules in a targeted fashion to the tumor or tumor microenvironment. Benefits of this delivery system can include reduction in the frequency of the dosages taken by a patient, a uniform effect of the drug, reduction of drug side-effects, and reduction of fluctuation in circulating drug levels.

Said moiety or TAM binding molecule or both can be held in a delivery agent. Said delivery agent can be a liposome, microsphere, nanoparticle, microemulsion, microcapsule, polymer matrix, hydrogel, or viral vector.

Liposomes are non-toxic, non-hemolytic, and non-immunogenic even upon repeated injections; they are biocompatible and biodegradable and can be designed to avoid clearance mechanisms (reticuloendothelial system (RES), renal clearance, chemical or enzymatic inactivation, or other undesirable effects. Lipid-based, ligand-coated nanocarriers can store their payload in a hydrophobic shell or a hydrophilic interior depending on the nature of the agent being carried.

Microspheres can encapsulate many types of drugs including small molecules, proteins, and nucleic acids and are easily administered through a syringe needle. Microspheres are generally biocompatible, can provide high bioavailability, and are capable of sustained release for long time periods. Disadvantages of microspheres include difficulty of large-scale manufacturing, inactivation of drug during fabrication, and poor control of drug release rates.

Nanoparticle-based drug delivery systems are contemplated to be a useful method for delivering agents. Some benefits of using nanoparticles include controllable release of the payload into the cytoplasm and circumvention of tumor drug resistance.

Microemulsions are isotropic, thermodynamically stable transparent (or translucent) systems of oil, water and surfactant, frequently in combination with a cosurfactant with a droplet size usually in the range of 20-200 nm. Said microemulsions can be classified as oil-in-water (o/w), water-in-oil (w/o) or bicontinuous systems depending on their structure and are characterized by ultra-low interfacial tension between oil and water phases. Said systems are currently of great technological and scientific interest to the researchers because of their potential to incorporate a wide range of drug molecules (hydrophilic and hydrophobic) due to the presence of both lipophilic and hydrophilic domains. These adaptable delivery systems provide protection against oxidation, enzymatic hydrolysis and improve the solubilization of lipophilic drugs and hence enhance their bioavailability. Microemulsions are suitable for oral and intravenous delivery systems and for sustained and targeted delivery (e.g. through ophthalmic, dental, pulmonary, vaginal and topical routes). Microemulsions have been used to improve the oral bioavailability of various poorly soluble drugs. In some instances, microemulsions are employed for challenging tasks such as carrying chemotherapeutic agents to neoplastic cells and oral delivery of insulin.
Microcapsules are particles with a diameter of 1-1000 µm, irrespective of the precise interior or exterior structure, which can be used for agent delivery. Microcapsules offer various significant advantages as drug delivery systems (e.g. an effective protection of the encapsulated active agent against (e.g. enzymatic) degradation, the possibility to accurately control the release rate of the incorporated drug over periods of hours to months, an easy administration (compared to alternative parenteral controlled release dosage forms, such as macro-sized implants), and desired, pre-programmed drug release profiles can be provided which match the therapeutic needs of the patient.

Agents can be embedded into a polymeric matrix or co-crystallized with a polymeric template. Polymers are a drug delivery technology which provide controlled release of therapeutic agents in constant doses over long periods, cyclic dosage, and tunable release of both hydrophilic and hydrophobic drugs. Polymer Matrices may be tailored for specific cargo and engineered to exert distinct biological functions.

Hydrogels are three-dimensional, cross-linked networks of water-soluble polymers. Hydrogels can be made from virtually any water-soluble polymer, encompassing a wide range of chemical compositions and bulk physical properties. Furthermore, hydrogels can be formulated in a variety of physical forms, including slabs, microparticles, nanoparticles, coatings, and films. As a result, hydrogels are commonly used in clinical practice and experimental medicine for a wide range of applications, including tissue engineering and regenerative medicine, diagnostics, cellular immobilization, separation of biomolecules or cells, and barrier materials to regulate biological adhesions

Virus delivery vectors are a type of nanomaterial which can be a drug delivery material. A successful vector must be able to effectively carry and subsequently deliver a drug cargo to a specific target.

Said conjugation can be achieved directly or indirectly, and with or without a linker molecule. This linker molecule can be a pH sensitive linker, a disulfide linker, a peptide linker, a beta-glucoronide linker, a redox responsive linker, a hydrazone linker, a hydrophilic linker, an azo linker, or another type of linker. Said linker can respond to a stimulus to initiate drug release. Said stimulus can be internal or external. Said stimulus can be local. Said stimulus can be pH, enzyme, light, heat, or another stimulus.

If the TAM binding molecule is conjugated to a moiety which is a therapeutic agent, said therapeutic agent can be a cytotoxic agent (e.g. ribosome inactivating protein, histone deacetylase inhibitor, tubulin inhibitor, alkylating agent, antibiotic, antineoplastic agent, antiproliferative agent, antimetabolite, topoisomerase I or II inhibitor, hormonal agonist or antagonist, immunomodulator, DNA minor groove binder or radioactive agent), a chemotherapeutic agent (e.g. alkylating agent, anthracycline, taxane, epothilone , topoisomerase I or II inhibitor, histone deacetylase inhibitor, kinase inhibitor, nucleotide analog, peptide antibiotic, platinum based agent, retinoid, vinca alkaloid or derivative, or other chemotherapeutic agent), a protein, a peptide, an antibody, a growth inhibitory agent, a nucleic acid, or an anti-hormonal agent.

If the TAM binding molecule is conjugated to a label, said label can be a fluorescent label, a chromogenic label, or a radiolabel. Said label is contemplated to be used for diagnostic imaging (e.g. PET, or MRI imaging or an imaging protocol incorporating PET or MRI imaging).

### EXAMPLES

### Example 1: CRV specifically binds to macrophages in vitro

RAW (tumor-derived mouse macrophage cell line RAW264.7), J774 (tumor-derived mouse macrophage cell line J774A.1), THP-1 differentiated macrophages (human macrophages differentiated from the human monocytic cell line THP-1) and 4T1 (mouse breast cancer cell line) cells were cultured in DMEM supplemented with 10% FCS. 1×10⁶ cells were incubated with FAM-CRV or the control peptide FAM-GGS (10 µM) in 300 µL of complete growth medium in an Eppendorf tube. After incubation at 4 °C for 1 h, the peptide-containing medium was removed by centrifugation and the cells were washed with PBS two times. 100 µL PFA (4% buffer) was then added to the cells for fixation and flow cytometry data were acquired on FACSCanto (BD Biosciences, San Jose). Experiment was repeated three times on different days.

FAM-CRV showed a much higher binding to RAW, J774, and THP-1 differentiated macrophages than did the control peptide FAM-GGS (Figure 1A). Contrarily, there was very limited binding of FAM-CRV to the 4T1 breast cancer cell line. These results indicated that CRV specifically binds to macrophages in vitro.

### Example 2: CRV binding to macrophages in vivo

To investigate if CRV can bind to macrophages in vivo, FAM-CRV was intravenously injected into tumor bearing mice. To this end, 4T1 and MCF10CA1a human breast cancer cells and KRAS-Ink mouse PDAC cells were cultured in Dulbecco's modified Eagle's medium supplemented with 10% fetal bovine serum, 100 U/mL penicillin and 100 µg/mL streptomycin. Py8119 cells were cultured in Ham's F12K medium containing 5% FCS, 2.5 µg/mL amphotericin B, 50 µg/mL gentamycin, and MITO+. The human cell lines were authenticated by the DNA Analysis Core Facility at the Sanford Burnham Prebys Medical Discovery Institute (La Jolla, CA) and the KRAS-Ink cell line was authenticated by DDC Medical (Fairfield, OH). All cells were tested negative for mycoplasma contamination. To produce 4T1 tumors, 1 × 10⁶ tumor cells (suspended in 100 µL of PBS) were orthotopically injected into the mammary fat pad of normal BALB/c mice. To produce MCF10CA1a tumors, 2 × 10⁶ tumor cells (suspended in 100 µL of PBS) were injected into the mammary fat pads of female BALB/c athymic nude mice. To produce Py8119 tumors, 1 × 10⁶ tumor cells (suspended in 100 µL of PBS) were orthotopically injected into the mammary fat pads of C57BL6 mice. To produce KRAS-Ink PDAC tumors, 1 × 10⁶ cells (suspended in 100 µL of PBS) were injected into female BALB/c mice. To produce H1975 tumors, 1x10⁶ cells (suspended in 100 µL of PBS) were injected. All animal experimentation received approval from the Animal Research Committee of Sanford Burnham Prebys Medical Discovery Institute.

Biodistribution of fluorescein-conjugated peptides (FAM-CRV, FAM-GGS, or FAM-ARA) was examined after intravenous injection of 100 µL peptide solution (1 mg/mL PBS) into the tail vein of a mouse. The peptide was allowed to circulate for 1 h and transcardial perfusion was performed with PBS. Tissues were collected, fixed with 4% formaldehyde buffer solution and then soaked in 30% sucrose in PBS overnight.

The tumor models tested in our study were summarized in Table 1. FAM-CRV homing was found positive in orthotopic 4T1 breast cancer, orthotopic MCF10CA1a breast cancer, subcutaneous kras-INK pancreatic cancer, subcutaneous KPC pancreatic cancer, and subcutaneous H1975 lung cancer. Compared to other organs, the tumors displayed a strong fluorescent signal under UV illumination.

**TABLE 1**

| Cell line | Tumor type/origin | Model/tumor site | In vivo homing |
|---|---|---|---|
| 4T1 | Mouse mammary carcinoma | Orthotopic mammary gland | Positive |
| MCF10CA1a | Mouse mammary carcinoma | Orthotopic mammary gland | Positive |
| Py8119 | Mouse mammary carcinoma | Orthotopic mammary gland | Negative |
| INK | Mouse pancreatic cancer | Subcutaneous | Positive |
| KPC | Mouse pancreatic ductal adenocarcinoma | Liver or Subcutaneous | Positive |
| H1975 | Human lung adenocarcinoma | Subcutaneous | Positive |

Representative images of FAM-CRV 1-h homing in the 4T1 breast cancer mouse model is shown in Figure 1B. FAM-CRV was mainly observed in the tumors and the kidneys, and modest to low accumulation was observed liver and spleen, which have high monocyte / macrophage content. Similar biodistribution was observed in the other CRV-positive models. These results suggested that FAM-CRV preferentially homes to tumors rather than other macrophage rich organs.

### Example 3: CRV homing within tumors

FAM-CRV was intravenously injected into tumor bearing mice. To this end, 4T1 human breast cancer cells were cultured in Dulbecco's modified Eagle's medium supplemented with 10% fetal bovine serum, 100 U/mL penicillin and 100 µg/mL streptomycin. The human cell line was authenticated by the DNA Analysis Core Facility at the Sanford Burnham Prebys Medical Discovery Institute (La Jolla, CA) and tested negative for mycoplasma contamination. To produce 4T1 tumors, 1 × 10⁶ tumor cells (suspended in 100 µL of PBS) were orthotopically injected into the mammary fat pad of normal BALB/c mice. All animal experimentation received approval from the Animal Research Committee of Sanford Burnham Prebys Medical Discovery Institute.

Biodistribution of fluorescein-conjugated peptides (FAM-CRV, FAM-GGS, or FAM-ARA) was examined after intravenous injection of 100 µL peptide solution (1 mg/mL PBS) into the tail vein of a mouse. The peptide was allowed to circulate for 1 h and transcardial perfusion was performed with PBS. Tissues were collected, fixed with 4% formaldehyde buffer solution and then soaked in 30% sucrose in PBS overnight. Tissues were finally frozen in OCT embedding medium (Tissue-Tek), and sliced for immunofluorescence staining.

Tissue sections were blocked in 1% bovine serum albumin with 0.1% Triton X-100 for 1 h, and incubated with appropriate primary antibodies and second antibodies. Blood vessels were visualized by staining tissue sections with monoclonal antibodies against CD-31. The primary antibody was rat anti-mouse CD31 (BD Biosciences). The secondary antibody was 594 donkey anti-rat IgG were from Invitrogen. After washing with PBS, sections were mounted in DAPI-containing mounting medium (Vector Laboratories, Burlingame, CA) and examined under a Zeiss LSM 710 NLO confocal microscope.

Immunofluorescence staining was performed for FAM-CRV and CD31 on all tissue sections. A representative image from 4T1 breast cancer model is shown in Figure 2. CRV homed to and spread within the tumors. Inside the tumors, the peptide can quickly leave the blood vessel within 5 min post administration as very little FAM-CRV signals colocalized with CD31 staining (Figure 2A). At different homing time points (5, 15, and 60 min), the signal of FAM-CRV gradually increased in the tumors. This indicated that the peptide penetrated across the blood vessels into the stroma. In comparison, the FAM-labeled control peptide, GGS (GGSGGSKG) yielded no fluorescence signals in the tumor at all time points. Interestingly it was found that FAM-CRV did accumulate in other organs, such as liver, spleen, and lymph nodes after 5 min circulation, but rapidly washed out after 1 h.

### Example 4: CRV targets macrophages in the tumor

To determine whether CRV targeted to macrophages or other cell types in the tumor, the tissue sections of 4T1 tumor-bearing mice were also stained for macrophages markers including CD11b, F4/80, and CD68 To this end, 4T1 human breast cancer cells were cultured in Dulbecco's modified Eagle's medium supplemented with 10% fetal bovine serum, 100 U/mL penicillin and 100 µg/mL streptomycin. The human cell line was authenticated by the DNA Analysis Core Facility at the Sanford Burnham Prebys Medical Discovery Institute (La Jolla, CA) and tested negative for mycoplasma contamination. To produce 4T1 tumors, 1 × 10⁶ tumor cells (suspended in 100 µL of PBS) were orthotopically injected into the mammary fat pad of normal BALB/c mice. All animal experimentation received approval from the Animal Research Committee of Sanford Burnham Prebys Medical Discovery Institute.

Biodistribution of fluorescein-conjugated peptides (FAM-CRV, FAM-GGS, or FAM-ARA) was examined after intravenous injection of 100 µL peptide solution (1 mg/mL PBS) into the tail vein of a mouse. The peptide was allowed to circulate for 1 h and transcardial perfusion was performed with PBS. Tissues were collected, fixed with 4% formaldehyde buffer solution and then soaked in 30% sucrose in PBS overnight. Tissues were finally frozen in OCT embedding medium (Tissue-Tek), and sliced for immunofluorescence staining.

Tissue sections were blocked in 1% bovine serum albumin with 0.1% Triton X-100 for 1 h, and incubated with appropriate primary antibodies and second antibodies. The primary antibodies were rat anti-mouse CD11b (BD Biosciences), rat anti-mouse F4/80 monoclonal (BD Biosciences), and rabbit anti-fluorescein/Oregon Green (Invitrogen) polyclonal antibodies. The secondary antibodies, Alexa Fluor 488 goat anti-rabbit IgG and 594 donkey anti-rat IgG were from Invitrogen. After washing with PBS, sections were mounted in DAPI-containing mounting medium (Vector Laboratories, Burlingame, CA) and examined under a Zeiss LSM 710 NLO confocal microscope.

Mice with tumor size about 8 mm were euthanized under deep anesthesia (Avertin, lack of response to a toe pinch) by cervical dislocation. Target (tumor) and control (e.g., liver, spleen) tissues were collected and further dissociated to single cells. Tumor cells were dissociated using MACS tumor dissociation kit. Cells were then incubated with fluorescent-labeled CRV or other cell markers (CD11b, CD68, F4/80, FAB, EpCAM, CD31) at 4 °C for 1 h. Positive cells were quantified on BD LSRFORTESSA and data were analyzed using FCS Express Version 3 (De Novo Software).

FAM-CRV signals strongly overlapped with those macrophages markers as shown in Figure 3A. This suggested that the accumulation of FAM-CRV in the tumor was mainly due to its association with tumor macrophages. Immunofluorescence staining was also performed on other animal models and received the same results. To further validate, cells were also isolated from tumors, spleen, liver, and blood from the 4T1 tumor mice and incubated with FAM-CRV for ex vivo binding studies. In the 4T1 model, tumor cells demonstrated much higher FAM-CRV binding compared to the spleen and blood cells, confirming a preferential homing of FAM-CRV into the tumor (Figure 3B). In comparison, the control FAM-GGS peptide could not specifically label macrophages in the tumor (Figure 3C). There was about 15% of the single tumor cells that showed very high FAM-CRV signals compared to the rest tumor cells, which were designated as FAM-CRV positive. More than 50% of FAM-CRV-positive cells were stained positive for both CD11b and F4/80 (Figure 3D). The low CRV-binding to the blood and splenic cells also suggested that FAM-CRV did not home to tumors by binding to circulating monocytes/macrophages, but recognized the tumor vasculatures, extravasated, and bound to macrophages in the tumor tissue.

### Example 5: FAM-CRV binds preferentially to tumor-associated macrophages

We further carried out flow cytometry analysis of dissociated 4T1 tumor cells after FAM-CRV in vivo homing (15, or 60 min post intravenous administration). To this end, FAM-CRV was intravenously injected into tumor bearing mice To this end, 4T1 human breast cancer cells were cultured in Dulbecco's modified Eagle's medium supplemented with 10% fetal bovine serum, 100 U/mL penicillin and 100 µg/mL streptomycin. The human cell line was authenticated by the DNA Analysis Core Facility at the Sanford Burnham Prebys Medical Discovery Institute (La Jolla, CA) and tested negative for mycoplasma contamination. To produce 4T1 tumors, 1 × 10⁶ tumor cells (suspended in 100 µL of PBS) were orthotopically injected into the mammary fat pad of normal BALB/c mice. All animal experimentation received approval from the Animal Research Committee of Sanford Burnham Prebys Medical Discovery Institute.

Fluorescein-conjugated peptides (100 µL, 1 mg/mL PBS) were intravenously injected into the tail vein of tumor-bearing mice. The peptide was allowed to circulate for 1 h. Mice were then euthanized under deep anesthesia (Avertin, lack of response to a toe pinch) by cervical dislocation. Tumors were collected and further dissociated to single cells, and incubated with different cell markers (CD11b, CD68, F4/80, FAB, EpCAM, CD31) at 4 °C for 1 h. Positive cells were quantified on BD LSRFORTESSA and data were analyzed using FCS Express Version 3 (De Novo Software).

The tumor cells from animals with both circulation lengths exhibited higher FAM-CRV signal compared to the non-injected control animals (Figure 3E). About 20% of the FAM-CRV positive cells were identified as macrophages, which was much less than the percentage in the ex vivo binding studies. This was probably due to the massive staining and washing steps for primary and secondary antibodies that many weakly bound FAM-CRV dissociated. All these binding results indicated that FAM-CRV can preferentially bind to tumor-associated macrophages.

### Example 6: FAM-CRV does not bind specifically to macrophages in atherosclerotic plaques

As macrophages reside in other disease sites, such as atherosclerotic plaques, it is interesting to know whether CRV recognizes other pathological tissues containing macrophages. Therefore, in vivo homing of FAM-CRV was tested in ApoE -/- mice bearing atherosclerotic plaques in the aorta. Unlike LyP1 , which homes to and bind to the macrophages both in tumors and plaques through the p32 receptor, FAM-CRV did not home to aortic plaques upon systemic administration (Figure 4A), suggesting that CRV can distinguish TAMs from macrophages in the atherosclerotic plaques in vivo. Immunofluorescence staining of the aorta also observed no FAM-CRV accumulation in the plaques. Single cell suspension was also obtained from mouse aorta with atherosclerotic plaques and the ex vivo binding study confirmed that FAM-CRV does not bind specifically to the macrophages in the plaques compared to FAM-GGS (Figure 4B). This feature makes CRV a desirable peptide to target macrophages for disease-specific applications.

### Example 7: RXRB is responsible for CRV binding to macrophages

The difference between CRV and LyP-1 suggests that CRV probably binds to a different receptor for TAM recognition. Therefore, the CRV receptor on macrophages was identified. To reduce the complexity, the membrane fraction of RAW cells was isolated, and performed affinity chromatography was performed to isolate putative CRV receptors. To this end, CRV was immobilized on a column. Lysates of 4T1 tumor tissue and membrane proteins of RAW cells (isolated with the Mem-PER Plus kit from Thermo Fisher) were utilized as two independent sources of putative receptors. After washing with buffer and the control peptide GGS, the putative receptor was eluted with an excess of free CRV peptide. Mass spectrometry analysis then identified the retinoid X receptor beta (RXRB) as a receptor candidate.

To confirm RXRB binds CRV, the binding of FAM-CRV to human recombinant RXRB protein immobilized on a plate was tested. Human RXRB recombinant protein (50 µL, 5 µg/mL) was immobilized in high binding 96-well plate at 4 °C overnight. Bovine serum albumin was used as the control protein. The protein solution was removed and washed one time with PBS. 1% BSA in PBS solution was then added to the wells and incubated at room temperature for 1 h to block the rest available binding sites. After one wash with PBS, RXRB and BSA were incubated with FAM-CRV (100 µL, 1 µM) at room temperature for 1 h. The wells were washed three times with PBS and the fluorescence was measured using a multiplate reader.

Compared to the control peptide FAM-GGS, there was significantly higher binding of CRV peptide to RXRB (Figure 5A). On the other hand, neither peptide bound to the control protein BSA. Both J774 and RAW cells displayed high surface expression of RXRB, as well as high FAM-CRV binding, while tumor cell lines such as 4T1 and PPC1 have very limited RXRB surface expression and low FAM-CRV binding. CRISPR was employed to silence the expression of RXRB in RAW cells and evaluated the binding of CRV peptide to those engineered cells. As shown in Figure 5B, silencing the expression of RXRB reduced about 70% of FAM-CRV binding to wild type RAW cells, which was quantified by flow cytometry after 1 h incubation. These in vitro results indicated that RXRB is responsible for the binding of CRV to macrophages.

### Example 8: RXRB surface binding is important for CRV binding

In addition, RXRB immunohistochemical (IHC) staining on 4T1 tumor tissues was performed. To this end, 4T1 human breast cancer cells were cultured in Dulbecco's modified Eagle's medium supplemented with 10% fetal bovine serum, 100 U/mL penicillin and 100 µg/mL streptomycin. The human cell line was authenticated by the DNA Analysis Core Facility at the Sanford Burnham Prebys Medical Discovery Institute (La Jolla, CA) and tested negative for mycoplasma contamination. To produce 4T1 tumors, 1 × 10⁶ tumor cells (suspended in 100 µL of PBS) were orthotopically injected into the mammary fat pad of normal BALB/c mice. All animal experimentation received approval from the Animal Research Committee of Sanford Burnham Prebys Medical Discovery Institute.

Tissue sections were blocked in 1% bovine serum albumin with 0.1% Triton X-100 for 1 h, and incubated with appropriate primary antibodies and second antibodies. The primary antibodies were rat anti-mouse CD11b (BD Biosciences), rat anti-mouse F4/80 monoclonal (BD Biosciences), and rabbit anti-fluorescein/Oregon Green (Invitrogen) polyclonal antibodies. The secondary antibodies, Alexa Fluor 488 goat anti-rabbit IgG and 594 donkey anti-rat IgG were from Invitrogen. After washing with PBS, sections were mounted in DAPI-containing mounting medium (Vector Laboratories, Burlingame, CA) and examined under a Zeiss LSM 710 NLO confocal microscope.

This experiment showed that RXRB co-localized with FAM-CRV as well as the macrophage markers (CD11b, F4/80, and CD68) (Figure 5C). It is also notable that the RXRB staining was presented on whole cells, not only on the nucleus, supporting that the RXRB surface expression was important for CRV binding.

### Example 9: RXRB positive cells have enhanced CRV binding

To further investigate the correlation between CRV binding to macrophages and the surface expression of RXRB, ex vivo binding of CRV and RXRB antibody to dissociated tumor cells from 4T1 tumor models was performed. Mice with tumor size about 8 mm were euthanized under deep anesthesia (Avertin, lack of response to a toe pinch) by cervical dislocation. Target (tumor) and control (e.g., liver, spleen) tissues were collected and further dissociated to single cells. Tumor cells were dissociated using MACS tumor dissociation kit. Cells were then incubated with fluorescent-labeled CRV or other cell markers (CD11b, F4/80) at 4 °C for 1 h. Positive cells were quantified on BD LSRFORTESSA and data were analyzed using FCS Express Version 3 (De Novo Software).

Flow cytometry analyses showed that about 15% cells from the tumor were RXRB positive and FAM-CRV primarily bound to cells with high RXRB surface expression (Figure 5D). The majority (about 73%) of the RXRB positive cells were CD11b and F4/80 positive (Figure 5E), indicating they were mainly expressed by TAMs. 73% of the FAM-CRV positive tumor cells were CD11b and RXRB positive. Staining of tumor cells after FAM-CRV 1h in vivo homing also demonstrated that the RXRB positive cells had higher CRV binding (Figure 5G). However, the difference was reduced compared to the ex vivo binding result (Figure 5D) due to FAM-CRV lost in the multiple washing steps.

### Example 10: Major organs have different levels of RXRB expression

The good correlation of RXRB and CRV lead us to the interest to investigate the surface expression of RXRB in different tissues, which could potentially explain the biodistribution of CRV in vivo. RXRB antibody (or Rabbit IgG as control) and FAM-CRV was both intravenously injected (RXRB antibody: 4 h circulation; FAM-CRV: 1 h circulation) to the same mice, and the presentation of RXRB was assessed after transcardial perfusion. Tissue sections were blocked in 1% bovine serum albumin with 0.1% Triton X-100 for 1 h, and incubated with appropriate primary antibodies and second antibodies. Blood vessels were visualized by staining tissue sections with monoclonal antibodies against CD-31. The primary antibodies were rat anti-mouse CD31 (BD Biosciences), rat anti-mouse CD11b (BD Biosciences), rat anti-mouse F4/80 monoclonal (BD Biosciences), and rabbit anti-fluorescein/Oregon Green (Invitrogen) polyclonal antibodies. The secondary antibodies, Alexa Fluor 488 goat anti-rabbit IgG and 594 donkey anti-rat IgG were from Invitrogen. After washing with PBS, sections were mounted in DAPI-containing mounting medium (Vector Laboratories, Burlingame, CA) and examined under a Zeiss LSM 710 NLO confocal microscope.

Immunofluorescence staining of the tissue sections showed that major organs had different levels of RXRB expression. RXRB can be found in tumor, liver, spleen and kidney, but not in heart and lung, while the IgG control staining was not observable in any organs. FAM-CRV signal can only be identified in tumor and kidney after its 1 h circulation. In the tumor, where RXRB staining was the highest among all the organs, RXRB showed nice colocalization with FAM-CRV and macrophage markers, best with CD11b, followed by CD68, and then F4/80 (Figure 6). In the liver, RXRB expression was much less and mainly colocalized with CD31 and little with CD68. As for the spleens, RXRB antibody was in part of red pulp area and did not have colocalization with CD31, and very little with CD68. The RXRB expression was very low and colocalized mainly with CD31 in the lymph nodes and kidneys. This could explain why FAM-CRV was quickly washed out in these two organs with time. Notably, the RXRB antibody staining results were similar with or without the Triton X-100 reagent for cell penetration.

### Example 11: RXRB is a surface marker with high specific expression on tumor macrophages

50 µg rabbit anti-mouse RXRB antibody (GeneTex, diluted in PBS, total volume was 100 µL) was intravenously injected to each mouse (n = 3). 50 µg rabbit IgG per mouse was injected to the control group (n = 3). Three hours later, FAM-CRV (100 µg in 100 µL) were injected to all animals. After 1 h circulation for FAM-CRV, animals were sacrificed with transcardial perfusion. Tissues were collected, fixed and sliced as described above.

When RXRB antibody was intravenously injected alone to 4T1 mice (also 4 h circulation), the apparent distribution of RXRB was the same as with FAM-CRV co-injection, indicating that the FAM-CRV presence did not affect the RXRB recognition by the antibody. In other words, RXRB antibody did not block CRV binding to RXRB in vivo, nor vice versa. This indicated that FAM-CRV bound to a different binding site as the RXRB antibody, which was in agreement as what was observed in the in vitro binding studies. These in vivo homing results demonstrated that RXRB is a good surface marker with its high specific expression on tumor macrophages. It also showed that RXRB antibody can preferentially home to tumors upon intravenous injection, penetrate the blood vessels and bind to macrophages in the extravascular regions, and function as a targeting agent.

### Example 12: CRV- mediated tumor homing of nanoparticles

To investigate the translational potential of the CRV-targeting approach, CRV-mediated tumor homing of nanoparticles was evaluated. There has been extensive works showing porous silicon nanoparticles (pSiNP) as therapeutic carriers with controlled loading and release of various types of drugs. Their high drug loading efficiency and time-gated imaging property make them particularly desirable as drug carriers.

As a proof of concept study, CRV-conjugated porous silicon nanoparticles (CRV-pSiNPs) were loaded with a red fluorescence probe, SR101 and the nanoparticles showed tumor homing effect in 4T1 tumor-bearing mice. 100-200 uL of a solution that contains CRV peptide, CRV-pSiNP, or control non-specific peptide was injected into the tail vein of tumor mice with a tumor size of about 8 mm. After 5 min, to up to 24 hours of circulation, the mice will be anesthetized and imaged with an imaging equipment (e.g., Xenogen IVIS, Lightools' light table, multiphoton LSM). When available as with Xenogen IVIS, the mice will be anesthetized with isoflurane during the imaging procedures (Induction: Flow rate 0.8 - 1.5 L/min, isoflurane vaporizer 2 - 3%, Maintenance: Flow rate 0.4 - 0.8 L/min, isoflurane vaporizer 2 - 3%). The mice will be then perfused with PBS. The tumors and control organs will be collected and used for histological characterization and immunohistochemical assessment.

The ex vivo IVIS imaging showed that there was higher SR101 intensity in the tumor of animals that received CRV-pSiNP compare to the control NP (Figure 7A). The tumor dissociated cells with flow cytometry analysis was also analyzed. In the CRV-pSiNP group, 74% of CRV-pSiNP positive cells were CD11b and CD68 positive, while only 52.5% of control pSiNP positive cells were CD11b and CD68 positive macrophages. This suggested that CRV targeted NPs mimic the homing ability of the free peptide and that CRV-pSiNP system has the potential to deliver drugs to tumor-associated macrophages, thus improving the clinical outcome. Thus, CRV-pSiNP can be loaded with drugs to tune macrophage polarity and evaluate the therapeutic effect on tumor treatment.

## Claims

1. A tumor associated macrophage (TAM) binding molecule for use in:
(a) a method of treating a cancer in a subject, the method comprising administering to the subject a pharmaceutical composition comprising the TAM binding molecule conjugated to a therapeutic agent; or
(b) a method of diagnosing a cancer in a subject, the method comprising administering to the subject a pharmaceutical composition comprising the TAM binding molecule conjugated to a label,
wherein the TAM binding molecule binds to retinoid X receptor beta on the TAM, and wherein the TAM binding molecule is an anti-RXR antibody, or antigen-binding fragment thereof, or the TAM binding molecule is CRVLRSGSC.

2. The TAM binding molecule for use according to claim 1, wherein:
(i) the antibody is an IgG, IgA, or IgM antibody; or
(ii) the antibody is a single domain antibody; or
(iii) the antibody is a chimeric, humanized, or human antibody; or
(iv) the antibody is a monoclonal antibody; or
(v) the antigen binding fragment is a Fab, Fab', Fab'-SH, Fv, scFv, F(ab')2, or a diabody.

3. The TAM binding molecule for use according to claim 1, wherein the therapeutic agent is a cytotoxic agent, a chemotherapeutic agent, a protein, a peptide, an antibody, a growth inhibitory agent, a nucleic acid or an anti-hormonal agent.

4. The TAM binding molecule for use according to claim 3, wherein the cytotoxic agent is a ribosome inactivating protein, a histone deacetylase (HDAC) inhibitor, a tubulin inhibitor, an alkylating agent, an antibiotic, an antineoplastic agent, an antiproliferative agent, an antimetabolite, a topoisomerase I or II inhibitor, a hormonal agonist or antagonist, an immunomodulator, a DNA minor groove binder, or a radioactive agent.

5. The TAM binding molecule for use according to claim 1, wherein the composition further comprises a delivery agent.

6. The TAM binding molecule for use according to claim 5, wherein the delivery agent comprises liposomes, microspheres, nanoparticles, microemulsions, microcapsules, polymer matrices, hydrogels, or viral vectors.

7. The TAM binding molecule for use according to claim 1, wherein the cancer is selected from the group consisting of brain cancer, renal cancer, ovarian cancer, prostate cancer, colon cancer, lung cancer, squamous cell carcinoma of head and neck, and melanoma.

8. The TAM binding molecule for use according to claim 1, wherein the pharmaceutical composition is administered subcutaneously, intravenously, intradermally, intraperitoneally, orally, intramuscularly or intracranially.

9. The TAM binding molecule for use according to claim 1, wherein the pharmaceutical composition is administered in combination with a second therapeutic agent.

10. The TAM binding molecule for use according to claim 9, wherein the second therapeutic agent is a cancer chemotherapeutic agent, radiation therapy, a cytotoxic agent, another antibody, a NSAID, a corticosteroid, a dietary supplement such as an antioxidant, or a combination thereof.

## Patentansprüche

1. Tumorassoziiertes Makrophagen (TAM)-bindendes Molekül zur Verwendung in:
(a) einem Verfahren zur Behandlung einer Krebserkrankung bei einem Patienten, wobei das Verfahren umfasst, dem Patienten eine pharmazeutische Zusammensetzung zu verabreichen, die das TAM-bindende Molekül, konjugiert mit einem therapeutischen Mittel, umfasst; oder
(b) einem Verfahren zur Diagnose einer Krebserkrankung bei einem Patienten, wobei das Verfahren umfasst, dem Patienten eine pharmazeutische Zusammensetzung zu verabreichen, die das TAM-bindende Molekül, konjugiert mit einem Marker, umfasst,
wobei das TAM-bindende Molekül an den Retinoid-X-Rezeptor Beta auf dem TAM bindet und
wobei das TAM-bindende Molekül ein Anti-RXR-Antikörper oder ein Antigenbindendes Fragment davon ist, oder das TAM-bindende Molekül CRVLRSGSC ist.

2. TAM-bindendes Molekül zur Verwendung nach Anspruch 1, wobei:
(i) der Antikörper ein IgG-, IgA- oder IgM-Antikörper ist; oder
(ii) der Antikörper ein Einzeldomänen-Antikörper ist; oder
(iii) der Antikörper ein chimärer, humanisierter oder humaner Antikörper ist; oder
(iv) der Antikörper ein monoklonaler Antikörper ist; oder
(v) das Antigenbindungsfragment ein Fab, Fab', Fab'-SH, Fv, scFv, F(ab')2 oder ein Diabody ist.

3. TAM-bindendes Molekül zur Verwendung nach Anspruch 1, wobei das therapeutische Mittel ein zytotoxisches Mittel, ein chemotherapeutisches Mittel, ein Protein, ein Peptid, ein Antikörper, ein wachstumshemmendes Mittel, eine Nukleinsäure oder ein antihormonelles Mittel ist.

4. TAM-bindendes Molekül zur Verwendung nach Anspruch 3, wobei das zytotoxische Mittel ein Ribosom-inaktivierendes Protein, ein Histon-Deacetylase-(HDAC)-Inhibitor, ein Tubulin-Inhibitor, ein Alkylierungsmittel, ein Antibiotikum, ein Antineoplastikum, ein Antiproliferativum, ein Antimetabolit, ein Topoisomerase-I- oder -II-Inhibitor, ein Hormonagonist oder -antagonist, ein Immunmodulator, ein DNA-Minor-Groove-Binder oder ein radioaktives Mittel ist.

5. TAM-bindendes Molekül zur Verwendung nach Anspruch 1, wobei die Zusammensetzung weiter ein Freisetzungsmittel umfasst.

6. TAM-bindendes Molekül zur Verwendung nach Anspruch 5, wobei das Freisetzungsmittel Liposomen, Mikrokügelchen, Nanopartikel, Mikroemulsionen, Mikrokapseln, Polymermatrizen, Hydrogele oder virale Vektoren umfasst.

7. TAM-bindendes Molekül zur Verwendung nach Anspruch 1, wobei der Krebs aus der Gruppe ausgewählt ist, die aus Hirnkrebs, Nierenkrebs, Eierstockkrebs, Prostatakrebs, Darmkrebs, Lungenkrebs, Plattenepithelkarzinom des Kopfes und Halses und Melanom besteht.

8. TAM-bindendes Molekül zur Verwendung nach Anspruch 1, wobei die pharmazeutische Zusammensetzung subkutan, intravenös, intradermal, intraperitoneal, oral, intramuskulär oder intrakraniell verabreicht wird.

9. TAM-bindendes Molekül zur Verwendung nach Anspruch 1, wobei die pharmazeutische Zusammensetzung in Kombination mit einem zweiten therapeutischen Mittel verabreicht wird.

10. TAM-bindendes Molekül zur Verwendung nach Anspruch 9, wobei das zweite therapeutische Mittel ein Krebs-Chemotherapeutikum, eine Strahlentherapie, ein Zytostatikum, ein anderer Antikörper, ein NSAID, ein Kortikosteroid, ein Nahrungsergänzungsmittel wie beispielsweise ein Antioxidans oder eine Kombination davon ist.

## Revendications

1. Molécule de liaison aux macrophages associés aux tumeurs (TAM) destinée à être utilisée dans :
(a) un procédé de traitement d'un cancer chez un sujet, le procédé comprenant l'administration au sujet d'une composition pharmaceutique comprenant la molécule de liaison TAM conjuguée à un agent thérapeutique ; ou
(b) un procédé de diagnostic d'un cancer chez un sujet, le procédé comprenant l'administration au sujet d'une composition pharmaceutique comprenant la molécule de liaison TAM conjuguée à une étiquette,
dans laquelle la molécule de liaison TAM se lie au récepteur bêta du rétinoïde X sur les TAM, et
dans laquelle la molécule de liaison TAM est un anticorps anti-RXR ou un fragment de liaison à l'antigène de celui-ci, ou dans laquelle la molécule de liaison TAM est CRVLRSGSC.

2. Molécule de liaison TAM destinée à être utilisée selon la revendication 1, dans laquelle :
(i) l'anticorps est un anticorps IgG, IgA ou IgM ; ou
(ii) l'anticorps est un anticorps à domaine unique ; ou
(iii) l'anticorps est un anticorps chimérique, humanisé ou humain ; ou
(iv) l'anticorps est un anticorps monoclonal ; ou
(v) le fragment de liaison à l'antigène est un Fab, Fab', Fab'-SH, Fv, scFv, F(ab')2 ou un diabody.

3. Molécule de liaison TAM destinée à être utilisée selon la revendication 1, dans laquelle l'agent thérapeutique est un agent cytotoxique, un agent chimiothérapeutique, une protéine, un peptide, un anticorps, un agent inhibiteur de croissance, un acide nucléique ou un agent anti-hormonal.

4. Molécule de liaison TAM destinée à être utilisée selon la revendication 3, dans laquelle l'agent cytotoxique est une protéine inactivant les ribosomes, un inhibiteur d'histone désacétylase (HDAC), un inhibiteur de tubuline, un agent alkylant, un antibiotique, un agent antinéoplasique, un agent antiprolifératif, un antimétabolite, un inhibiteur de topoisomérase I ou II, un agoniste ou un antagoniste hormonal, un immunomodulateur, un liant du sillon mineur de l'ADN, ou un agent radioactif.

5. Molécule de liaison TAM destinée à être utilisée selon la revendication 1, dans laquelle la composition comprend en outre un agent d'administration.

6. Molécule de liaison TAM destinée à être utilisée selon la revendication 5, dans laquelle l'agent d'administration comprend des liposomes, des microsphères, des nanoparticules, des microémulsions, des microcapsules, des matrices polymères, des hydrogels, ou des vecteurs viraux.

7. Molécule de liaison TAM destinée à être utilisée selon la revendication 1, dans laquelle le cancer est choisi dans le groupe constitué par le cancer du cerveau, le cancer du rein, le cancer de l'ovaire, le cancer de la prostate, le cancer du côlon, le cancer du poumon, le carcinome épidermoïde de la tête et du cou, et le mélanome.

8. Molécule de liaison TAM destinée à être utilisée selon la revendication 1, dans laquelle la composition pharmaceutique est administrée par voie sous-cutanée, intraveineuse, intradermique, intrapéritonéale, orale, intramusculaire ou intracrânienne.

9. Molécule de liaison TAM destinée à être utilisée selon la revendication 1, dans laquelle la composition pharmaceutique est administrée en association avec un second agent thérapeutique.

10. Molécule de liaison TAM destinée à être utilisée selon la revendication 9, dans laquelle le second agent thérapeutique est un agent chimiothérapeutique anticancéreux, une radiothérapie, un agent cytotoxique, un autre anticorps, un AINS, un corticostéroïde, un complément alimentaire tel qu'un antioxydant, ou une combinaison de ceux-ci.
